# EUROPEAN PATENT APPLICATION

(11) **EP 2 857 032 A2**
(43) Date of publication of application: **08.04.2015**
(21) Application number: 14003460.4
(22) Date of filing: 28.10.2011
(51) Int. Cl.: A61K 36/73, A61K 36/00, A61K 36/18, A61K 36/28, A61P 9/00, A61P 43/00

(54) **Tie2 activator, vascular endothelial growth factor (vegf) inhibitor, anti-angiogenic agent, agent for maturing blood vessels, agent for normalizing blood vessels, agent for stabilizing blood vessels, and pharmaceutical composition**

(30) Priority: 02.12.2010 JP 2010269800; 10.05.2011 JP 2011105696; 10.05.2011 JP 2011105695; 08.07.2011 JP 2011151593
(62) Divisional of application: 11845773.8
(71) Applicant: Maruzen Pharmaceuticals Co., Ltd., Onomichi-shi Hiroshima 722-0062 (JP)
(72) Inventor: Ohto, Nobuaki, Fukuyama-shi Hiroshima, 729-3102 (JP); Kiso, Akinori, Fukuyama-shi Hiroshima, 729-3102 (JP)
(74) Representative: Graf von Stosch, Andreas

(57) **Abstract**

To provide a Tie2 activator, a vascular endothelial growth factor (VEGF) inhibitor, an angiogenesis inhibitor, a vascular maturing agent, a vascular normalizing agent and a vascular stabilizing agent, and a pharmaceutical composition having high safety, and an excellent Tie2 activating effect, vascular endothelial growth factor (VEGF) inhibitory effect, angiogenesis inhibitory effect, vascular maturing effect, vascular normalizing effect, and vascular stabilizing effect.

A Tie2 activator, a vascular endothelial growth factor (VEGF) inhibitor, an angiogenesis inhibitor, a vascular maturing agent, a vascular normalizing agent and a vascular stabilizing agent, and a pharmaceutical composition containing, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.

## Description

### Technical Field

The present invention relates to a Tie2 activator, a vascular endothelial growth factor (VEGF) inhibitor, an angiogenesis inhibitor, a vascular maturing agent, a vascular normalizing agent, and vascular stabilizing agent, and a pharmaceutical composition.

### Background Art

Blood vessels have a structure in which vascular endothelial cells adhere to vascular wall cells (vascular smooth muscle cells and pericytes) either directly or indirectly via extracellular matrix, and have a function to supply oxygen and nutrients to living tissues and to remove wastes from living tissues.

Generally, blood vessel formation is divided into two steps: vasculogenesis by which new blood vessels are formed; and angiogenesis by which a new vascular network is formed by elongation and branching of existing blood vessels. The former is involved in an extremely wide range of blood vessel formation ranging from initial formation of blood vessels referred to as angiopoiesis to subsequent angiogenesis by the action of a vascular endothelial growth factor (VEGF). Meanwhile, the latter is involved in controlling adhesion between vascular endothelial cells and vascular wall cells and in structural stabilization of blood vessels by the action of angiopoietin (Ang).

Under normal oxygen condition, vascular endothelial cells and vascular wall cells lining therearound are tightly adhered to each other to thereby maintain a stable structure of blood vessels. On the other hand, under hypoxic condition, vascular wall cells are detached from vascular endothelial cells, potentially leading to an unregulated vascular growth. Such phenomenon (angiogenesis) has been often observed in diseases with vascular lesions such as tumors, chronic rheumatoid arthritis, diabetic retinopathy, hyperlipemia or hypertension.

The angiogenesis has been known to be inhibited by activating a type of receptor tyrosine kinase Tie2 (Tyrosine kinase with Ig and EGF homology domain 2) which expresses in vascular endothelial cells (for example, see PTL 1). In ischemic diseases caused by vascular narrowing or inhibition of vascular expansion, it has been reported that vascular lumens are expanded by an activation of Tie2 (for example, see NPL 1). In addition, the activation of Tie2 has been reported to inhibit cell death of vascular endothelial cells (for example, see NPL 2).

In addition to inhibiting the angiogenesis as described above, the activation of Tie2 has been known to mature, normalize, and stabilize blood vessels.

For example, in revascularization therapy, it has been known that the activation of Tie2 induces adhesion between vascular endothelial cells and vascular wall cells to thereby mature blood vessels.

For example, in diseases such as tumors or diabetic retinopathy characterized by unregulated vascular growth caused by detachment of vascular wall cells from vascular endothelial cells, it has been known that the activation of Tie2 makes the vascular wall cells adhere to the vascular endothelial cells to thereby normalize blood vessels.

For example, it has been known that the activation of Tie2 inhibits destabilization of blood vessels due to environmental factors that damage various intracellular and extracellular vascular structures to thereby stabilize blood vessels.

A cinnamon extract has been known as a naturally occurring material having a function to inhibit the angiogenesis by the activation of Tie2 (for example, see PTL 1). However, the cinnamon extract has a disadvantage of unsatisfactory activity. In addition, suramin has also been known as a material having a function to inhibit the angiogenesis (for example, see PTL 2). However, suramin has a disadvantage of low safety.

Therefore, at present, keen demand has arisen for development for a highly safe material having an excellent Tie2 activating effect, vascular endothelial growth factor (VEGF) inhibitor, angiogenesis inhibitory effect, vascular maturing effect, vascular normalizing effect, and vascular stabilizing effect.

### Citation List

### Patent Literature

PTL 1 Japanese Patent Application Laid-Open (JP-A) No. 2009-263358
PTL 2 JP-A No. 09-503488

### Non Patent Literature

NPL 1 Science. 1999. Dec. 24; 286 (5449) 2511-4.
NPL 2 P. N. A. S. 2004. Apr. 13; 101 (15) 5553-8.

### Summary of the Invention

### Technical Problem

The present invention aims to solve the above existing problems and achieve the following objects. An object of the present invention is to provide a Tie2 activator having an excellent Tie2 activating effect and high safety. Also, an object of the present invention is to provide a vascular endothelial growth factor (VEGF) inhibitor having an excellent vascular endothelial growth factor (VEGF) inhibitory effect and high safety. Also, an object of the present invention is to provide an angiogenesis inhibitor having an excellent angiogenesis inhibitory effect and high safety. Also, an object of the present invention is to provide a vascular maturing agent having an excellent vascular maturing effect and high safety. Also, an object of the present invention is to provide a vascular normalizing agent having an excellent vascular normalizing effect and high safety. Also, an object of the present invention is to provide a vascular stabilizing agent having an excellent vascular stabilizing effect and high safety. Also, an object of the present invention is to provide a pharmaceutical composition having an excellent Tie2 activating effect, vascular endothelial growth factor (VEGF) inhibitory effect, angiogenesis inhibitory effect, vascular maturing effect, vascular normalizing effect, or vascular stabilizing effect, or any combination thereof, and high safety.

### Solution to Problem

The present inventors conducted extensive studies to achieve the above objects, and have found that the following extracts have the excellent Tie2 activating effect, vascular endothelial growth factor (VEGF) inhibitory effect, angiogenesis inhibitory effect, vascular maturing effect, vascular normalizing effect, and vascular stabilizing effect. Thus, the present invention has been accomplished.

The present invention is based on the above finding obtained by the present inventors. Means for solving the above problems are as follows.
<1> A Tie2 activator containing, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.
<2> An angiogenesis inhibitor containing, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.
<3> A vascular maturing agent, a vascular normalizing agent or a vascular stabilizing agent containing, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.
<4> A vascular endothelial growth factor (VEGF) inhibitor containing, as an active ingredient, a hawthorn extract.
<5> A pharmaceutical composition containing the Tie2 activator according to <1>, the angiogenesis inhibitor according to <2>, the vascular maturing agent, the vascular normalizing agent or the vascular stabilizing agent according to <3>, or the vascular endothelial growth factor (VEGF) inhibitor according to <4>, or any combination thereof.

### Advantageous Effects of Invention

According to the Tie2 activator of the present invention, the above existing problems can be solved and there can be provided a Tie2 activator having an excellent Tie2 activating effect and high safety.

According to the vascular endothelial growth factor (VEGF) inhibitor of the present invention, the above existing problems can be solved and there can be provided a vascular endothelial growth factor (VEGF) inhibitor having an excellent vascular endothelial growth factor (VEGF) inhibitory effect and high safety.

According to the angiogenesis inhibitor of the present invention, the above existing problems can be solved and there can be provided an angiogenesis inhibitor having an excellent angiogenesis inhibitory effect and high safety.

According to the vascular maturing agent, the vascular normalizing agent, and the vascular stabilizing agent of the present invention, the above existing problems can be solved and there can be provided a vascular maturing agent, a vascular normalizing agent, and a vascular stabilizing agent having an excellent vascular maturing effect, vascular normalizing effect, and vascular stabilizing effect, and high safety.

According to the pharmaceutical composition of the present invention, the above existing problems can be solved and there can be provided a pharmaceutical composition having an excellent Tie2 activating effect, vascular endothelial growth factor (VEGF) inhibitory effect, angiogenesis inhibitory effect, vascular maturing effect, vascular normalizing effect, or vascular stabilizing effect, or any combination thereof, and high safety.

### Brief Description of Drawings

FIG. 1 shows phosphorylated Tie2 in each sample detected by Western blot.
FIG. 2 shows phosphorylated Tie2 in each sample detected by Western blot.
FIG. 3 shows phosphorylated Tie2 in each sample detected by Western blot.
FIG. 4 shows phosphorylated Tie2 in each sample detected by Western blot.
FIG. 5 shows Tie2 phosphorylation in each sample detected by Western blot.
FIG. 6 shows Tie2 phosphorylation in each sample detected by Western blot.
FIG. 7 shows the number of vascular branching measured in each sample.

### Description of Embodiments

### (Tie2 activator, vascular endothelial growth factor (VEGF) inhibitor, angiogenesis inhibitor, vascular maturing agent, vascular normalizing agent and vascular stabilizing agent, and pharmaceutical composition)

A Tie2 activator, a vascular endothelial growth factor (VEGF) inhibitor, a vascular maturing agent, a vascular normalizing agent, a vascular stabilizing agent and an angiogenesis inhibitor, and a pharmaceutical composition of the present invention contain, as active ingredients, at least any of the below described extracts; and, if necessary, further contain other ingredients.

The Tie2 activator has a Tie2 activating effect, i.e., it converts Tie2 to its active form (phosphorylated Tie2) by phosphorylation. The activation of Tie2 causes autophosphorylation of an intracellular tyrosine kinase domain to thereby induce adhesion between vascular endothelial cells and vascular wall cells. In ischemic diseases caused by vascular narrowing or inhibition of vascular expansion, vascular lumens are expanded by the activation of Tie2. In addition, the activation of Tie2 inhibits cell death of vascular endothelial cells.

The vascular endothelial growth factor (VEGF) inhibitor has a VEGF inhibitory effect, i.e., it inhibits an action of VEGF. The VEGF is a glycoprotein having the molecular weight of 34 kDa to 46 kDa. The VEGF acts on vascular endothelial cells and facilitates cell growth, cell migration and the angiogenesis.

The angiogenesis inhibitor has an angiogenesis inhibitory effect, i.e., it inhibits formation of a new vascular network from existing blood vessels. Under hypoxic condition, the activation of Tie2 is transiently inhibited, leading to detachment of vascular wall cells from vascular endothelial cells. Thus detached vascular endothelial cells form a new vascular network. The angiogenesis inhibitor can inhibit unregulated vascular growth caused by the detachment of vascular wall cells from vascular endothelial cells.

The vascular maturing agent has a maturing effect, i.e., it induces adhesion between vascular endothelial cells and vascular wall cells to thereby form desmosome between vascular endothelial cells so as to prevent a tendency of extravascular leakage of intravascular environmental factors (cellular and humoral factors). In revascularization therapy, blood vessels can be matured by activating Tie2 to thereby induce adhesion between endothelial cells and vascular wall cells in blood vessels

The vascular normalizing agent has a normalizing effect, i.e., it returns blood vessels in which vascular permeability has been impaired or abnormal blood vessels that cause unregulated vascular growth to their normal state by enhancing adhesion between vascular endothelial cells and facilitating lining of vascular endothelial cells with vascular wall cells. Also, in diseases such as tumors or diabetic retinopathy characterized by unregulated vascular growth caused by detachment of vascular wall cells from vascular endothelial cells, blood vessels can be normalized by activating Tie2 to thereby make vascular wall cells adhere to vascular endothelial cells.

The vascular stabilizing agent has a stabilizing effect, i.e., it inhibits damage to existing blood vessels, detachment of vascular endothelial cells from each other, and detachment of vascular wall cells from vascular endothelial cells, as well as cell death of vascular endothelial cells. Also, blood vessels can be stabilized by activating Tie2 to thereby inhibit destabilization of blood vessels due to environmental factors that damage various intracellular and extracellular vascular structures.

The pharmaceutical composition is a composition containing the Tie2 activator, the vascular endothelial growth factor (VEGF) inhibitor, the angiogenesis inhibitor, the vascular maturing agent, the vascular normalizing agent, or the vascular stabilizing agent, or any combination thereof; and has the Tie2 activating effect, the angiogenesis inhibitory effect, the vascular maturing effect, the vascular normalizing effect, or the vascular stabilizing effect, or any combination thereof.

### <Hawthorn extract>

The hawthorn (scientific name: Crataegus cuneata) is a deciduous shrub belonging to the genus Crataegus of the family Rosaceae. The hawthorn can be easily obtained from south-central China. The hawthorn has stems 30 cm to 90 cm high and produces purple or white flowers from August to September. A medicinal part of the hawthorn is a fruit and it has been widely used as a digestant.

The hawthorn extract can be easily obtained by using methods commonly used in extraction of plants. The hawthorn extract is not particularly limited and may be appropriately selected depending on the intended purpose. For example, the hawthorn extract may be in the form of diluted extract liquid, concentrated extract liquid or dried product of the extract.

A method for extracting the hawthorn is not particularly limited and may be appropriately selected depending on the intended purpose. For example, the hawthorn can be extracted as follows. Firstly, the hawthorn, an extraction feed, is added to a processing tank containing an extraction solvent. Soluble components contained in the hawthorn are allowed to dissolve in the extraction solvent while appropriately stirring, if necessary. Subsequently, extraction residue is removed by filtration to thereby obtain the hawthorn extract.

An extraction part of the hawthorn is not particularly limited and may be appropriately selected depending on the intended purpose. Examples thereof include leaves, branches, trunks, barks, flowers and fruits. Among them, preferred are fruits and leaves.

For example, the extraction part of the hawthorn can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

An extraction condition of the hawthorn is not particularly limited and may be appropriately selected depending on the intended purpose. For example, extraction time is preferably 1 hour to 3 hours and extraction temperature is preferably from room temperature to 95°C.

The extraction solvent of the hawthorn is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the hawthorn is preferably used at room temperature or at a temperature equal to or lower than the boiling point of the solvent.

Among them, the extraction is preferably performed with the hot water.

The amount of the extraction solvent of the hawthorn is not particularly limited and may be appropriately selected depending on the intended purpose, but is preferably 5 times to 15 times the mass of the hawthorn as the extraction feed (on a mass ratio basis).

A method for purifying the hawthorn extract is not particularly limited and may be appropriately selected depending on the intended purpose. The hawthorn extract may be purified by, for example, a liquid-liquid distribution extraction, various chromatographies, and a membrane separation.

The concentration of the hawthorn extract is not particularly limited and may be appropriately selected depending on the intended purpose, but is preferably 50 µg/mL to 400 µg/mL, more preferably 100 µg/mL to 400 µg/mL.

### <Starfruit extract>

The starfruit (scientific name: Averrhoa carambola, Japanese name: Gorenshi) is an evergreen woody plant belonging to the genus Averrhoa of the family Oxalidaceae. The starfruit is native to the whole area of Southeast Asia, as well as tropical and subtropical regions such as southern China, Taiwan, Brazil, Guyana, Trinidad and Tobago, Florida and Hawaii, and can be easily obtained from these regions. Some starfruits produce sweet fruits, others produce sour fruits. The starfruit is eaten raw or utilized for jam, jelly, and pickles. Leaves of the starfruit are used as a preventive or therapeutic agent for wind-heat-type cold, acute gastroenteritis, difficult urination and postpartum edema.

An extraction part of the starfruit is not particularly limited and may be appropriately selected depending on the intended purpose. Examples thereof include leaves, stems, flowers, fruits and roots. Among them, preferred are leaves.

The extraction part of the starfruit can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The starfruit extract can be easily obtained by using methods commonly used in extraction of plants. The starfruit extract is not particularly limited and may be appropriately selected depending on the intended purpose. For example, the starfruit extract may be in the form of diluted extract liquid, concentrated extract liquid or dried product of the extract.

A method for extracting the starfruit is not particularly limited and may be appropriately selected depending on the intended purpose. For example, the starfruit can be extracted as follows. Firstly, the extraction part of the starfruit, an extraction feed, is added to a processing tank containing an extraction solvent. Soluble components contained in the starfruit are allowed to dissolve in the extraction solvent while appropriately stirring, if necessary. Subsequently, extraction residue is removed by filtration to thereby obtain the starfruit extract.

Extraction conditions (extraction time and extraction temperature) of the starfruit and the amount of the extraction solvent of the starfruit are not particularly limited and may be appropriately selected depending on the intended purpose.

The extraction solvent of the starfruit is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the starfruit is preferably used at room temperature or at a temperature equal to or lower than the boiling point of the solvent. Among them, the extraction is preferably performed with the hot water.

A method for purifying the starfruit extract is not particularly limited and may be appropriately selected depending on the intended purpose. The starfruit extract may be purified by, for example, a liquid-liquid distribution extraction, various chromatographies, and a membrane separation.

The concentration of the starfruit extract is not particularly limited and may be appropriately selected depending on the intended purpose, but is preferably 100 µg/mL to 400 µg/mL, more preferably 200 µg/mL to 400 µg/mL.

### <Shellflower extract>

The shellflower (scientific name: Alpinia speciosa, Japanese name: Getto) is an evergreen perennial herb belonging to the genus Alpinia of the family Zingiberaceae. The shellflower is native to the southern extremity of Kyushu, Okinawa, the coast of Ogasawara Islands, Taiwan, southern China, Southeast Asia and India, and can be easily obtained from these regions. The shellflower has plant height of 2 m to 3 m. Stems thereof are fascicled and erect. Leaves thereof are chartaceous, alternate in 2 rows, oval-shaped and lanceolate. Flowers thereof have large drooping racemes.

An extraction part of the shellflower is not particularly limited and may be appropriately selected depending on the intended purpose. Examples thereof include leaves, stems, flowers, fruits and roots. Among them, preferred are leaves.

The extraction part of the shellflower can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The shellflower extract can be easily obtained by using methods commonly used in extraction of plants. The shellflower extract is not particularly limited and may be appropriately selected depending on the intended purpose. For example, the shellflower extract may be in the form of diluted extract liquid, concentrated extract liquid or dried product of the extract.

A method for extracting the shellflower is not particularly limited and may be appropriately selected depending on the intended purpose. For example, the shellflower can be extracted as follows. Firstly, the extraction part of the shellflower, an extraction feed, is added to a processing tank containing an extraction solvent. Soluble components contained in the shellflower are allowed to dissolve in the extraction solvent while appropriately stirring, if necessary. Subsequently, extraction residue is removed by filtration to thereby obtain the shellflower extract.

Extraction conditions (extraction time and extraction temperature) of the shellflower and the amount of the extraction solvent of the shellflower are not particularly limited and may be appropriately selected depending on the intended purpose.

The extraction solvent of the shellflower is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the shellflower is preferably used at room temperature or at a temperature equal to or lower than the boiling point of the solvent. Among them, the extraction is preferably performed with the hot water.

A method for purifying the shellflower extract is not particularly limited and may be appropriately selected depending on the intended purpose. The shellflower extract may be purified by, for example, a liquid-liquid distribution extraction, various chromatographies, and a membrane separation.

The concentration of the shellflower extract is not particularly limited and may be appropriately selected depending on the intended purpose, but is preferably 50 µg/mL to 400 µg/mL, more preferably 200 µg/mL to 400 µg/mL.

### <Lotus extract>

The lotus (scientific name: Nelumbo nucifera, Japanese name: Hasu) is a perennial hydrophyte belonging to the genus Nelumbo of the family Nelumbonaceae. The shellflower is native to Indian subcontinent and therearound, and can be easily obtained from these regions. Stems of the lotus grow from underground stems in the soil towards the water surface. Leaves thereof float on top of the water surface, are water-repellent, have a round shape, and have a petiole at the center. The lotus has plant height of about 1 m.

An extraction part of the shellflower is not particularly limited and may be appropriately selected depending on the intended purpose. Examples thereof include leaves, stems, germs, flowers, fruits and roots. Among them, preferred are germs.

The extraction part of the lotus can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The lotus extract can be easily obtained by using methods commonly used in extraction of plants. The lotus extract is not particularly limited and may be appropriately selected depending on the intended purpose. For example, the lotus extract may be in the form of diluted extract liquid, concentrated extract liquid or dried product of the extract.

A method for extracting the lotus is not particularly limited and may be appropriately selected depending on the intended purpose. For example, the lotus can be extracted as follows. Firstly, the extraction part of the lotus, an extraction feed, is added to a processing tank containing an extraction solvent. Soluble components contained in the lotus are allowed to dissolve in the extraction solvent while appropriately stirring, if necessary. Subsequently, extraction residue is removed by filtration to thereby obtain the lotus extract.

Extraction conditions (extraction time and extraction temperature) of the lotus and the amount of the extraction solvent of the lotus are not particularly limited and may be appropriately selected depending on the intended purpose.

The extraction solvent of the lotus is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the lotus is preferably used at room temperature or at a temperature equal to or lower than the boiling point of the solvent. Among them, the extraction is preferably performed with the hot water.

A method for purifying the lotus extract is not particularly limited and may be appropriately selected depending on the intended purpose. The lotus extract may be purified by, for example, a liquid-liquid distribution extraction, various chromatographies, and a membrane separation.

The concentration of the lotus extract is not particularly limited and may be appropriately selected depending on the intended purpose, but is preferably 100 µg/mL to 400 µg/mL, more preferably 200 µg/mL to 400 µg/mL.

### <Rooibos extract>

The rooibos (scientific name: Aspalathus linearis (N. L. Barum.) R. Dahlgr (Leguminosae) is a dicotyledon belonging to the genus Aspalathus of the family Fabaceae. The rooibos is cultivated in Cederberg Mountains of South Africa, and can be easily obtained from this region. Dried product of fermented young leaves and branches thereof has been reported to be effective for spasmolysis, emesis, diarrhea and other slight stomach disorders, and as roborant. Rooibos tea has a sweet taste, contains no caffeine and a very low level of tannin, and has an antioxidative effect.

An extraction part of the rooibos is not particularly limited and may be appropriately selected depending on the intended purpose. Examples thereof include leaves, young leaves, branches, trunks, barks, flowers, fruits and roots. Among them, preferred are young leaves and branches. Particularly, preferred is dry powder of young leaves and branches (rooibos tea).

The extraction part of the rooibos can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The rooibos extract can be easily obtained by using methods commonly used in extraction of plants. The rooibos extract is not particularly limited and may be appropriately selected depending on the intended purpose. For example, the rooibos extract may be in the form of diluted extract liquid, concentrated extract liquid or dried product of the extract.

A method for extracting the rooibos is not particularly limited and may be appropriately selected depending on the intended purpose. For example, the rooibos can be extracted as follows. Firstly, the extraction part of the rooibos, an extraction feed, is added to a processing tank containing an extraction solvent. Soluble components contained in the rooibos are allowed to dissolve in the extraction solvent while appropriately stirring, if necessary. Subsequently, extraction residue is removed by filtration to thereby obtain the rooibos extract.

Extraction conditions (extraction time and extraction temperature) of the rooibos and the amount of the extraction solvent of the rooibos are not particularly limited and may be appropriately selected depending on the intended purpose.

The extraction solvent of the rooibos is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the rooibos is preferably used at room temperature or at a temperature equal to or lower than the boiling point of the solvent. Among them, the extraction is preferably performed with the hot water.

A method for purifying the rooibos extract is not particularly limited and may be appropriately selected depending on the intended purpose. The rooibos extract may be purified by, for example, a liquid-liquid distribution extraction, various chromatographies, and a membrane separation.

The concentration of the rooibos extract is not particularly limited and may be appropriately selected depending on the intended purpose, but is preferably 50 µg/mL to 400 µg/mL, more preferably 200 µg/mL to 400 µg/mL.

### <Indian date extract>

The Indian date (scientific name: Tamarindus indica L.) is an evergreen tree belonging to the genus Tamarindus of the family Caesalpiniaceae. The Indian date is also called as tamarind. The Indian date grows naturally in tropical regions such as Asia and Africa, and is cultivated in, for example, India and Thailand. The Indian date can be easily obtained from these regions. In Japan, polysaccharides (tamarind seed gum) contained in endosperm portions of the Indian date seeds have been utilized for food as, for example, thickeners. Also, tannin contained in seed coats thereof has been utilized as dyes.

An extraction part of the Indian date is not particularly limited and may be appropriately selected depending on the intended purpose. Examples thereof include leaves, branches, trunks, barks, flowers, fruits, seeds, seed coats and roots. Among them, preferred are seed coats.

The extraction part of the Indian date can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Indian date extract can be easily obtained by using methods commonly used in extraction of plants. The Indian date extract is not particularly limited and may be appropriately selected depending on the intended purpose. For example, the Indian date extract may be in the form of diluted extract liquid, concentrated extract liquid or dried product of the extract.

A method for extracting the Indian date is not particularly limited and may be appropriately selected depending on the intended purpose. For example, the Indian date can be extracted as follows. Firstly, the extraction part of the Indian date, an extraction feed, is added to a processing tank containing an extraction solvent. Soluble components contained in the Indian date are allowed to dissolve in the extraction solvent while appropriately stirring, if necessary. Subsequently, extraction residue is removed by filtration to thereby obtain the Indian date extract.

Extraction conditions (extraction time and extraction temperature) of the Indian date and the amount of the extraction solvent of the Indian date are not particularly limited and may be appropriately selected depending on the intended purpose.

The extraction solvent of the Indian date is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Indian date is preferably used at room temperature or at a temperature equal to or lower than the boiling point of the solvent. Among them, the extraction is preferably performed with the hot water.

A method for purifying the Indian date extract is not particularly limited and may be appropriately selected depending on the intended purpose. The Indian date extract may be purified by, for example, a liquid-liquid distribution extraction, various chromatographies, and a membrane separation.

The concentration of the Indian date extract is not particularly limited and may be appropriately selected depending on the intended purpose, but is preferably 10 µg/mL to 100 µg/mL, more preferably 10 µg/mL to 20 µg/mL.

### <Chinese quince extract>

The Chinese quince (scientific name: Chaenomeles sinensis) is a deciduous tree belonging to the genus Chaenomeles of the family Rosaceae. The Chinese quince is native to eastern China, is cultivated in, for example, Japan, China, Korea, America and France, and can be easily obtained from these regions. Fruits of the Chinese quince are called as wamokka, a crude drug name. From ancient times, they have been widely utilized as a cough suppressant for reducing inflammation of throat.

An extraction part of the Chinese quince is not particularly limited and may be appropriately selected depending on the intended purpose. Examples thereof include leaves, branches, trunks, barks, flowers and fruits. Among them, preferred are fruits.

The extraction part of the Chinese quince can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Chinese quince extract can be easily obtained by using methods commonly used in extraction of plants. The Chinese quince extract is not particularly limited and may be appropriately selected depending on the intended purpose. For example, the Chinese quince extract may be in the form of diluted extract liquid, concentrated extract liquid or dried product of the extract.

A method for extracting the Chinese quince is not particularly limited and may be appropriately selected depending on the intended purpose. For example, the Chinese quince can be extracted as follows. Firstly, the extraction part of the Chinese quince, an extraction feed, is added to a processing tank containing an extraction solvent. Soluble components contained in the Chinese quince are allowed to dissolve in the extraction solvent while appropriately stirring, if necessary. Subsequently, extraction residue is removed by filtration to thereby obtain the Chinese quince extract.

Extraction conditions (extraction time and extraction temperature) of the Chinese quince and the amount of the extraction solvent of the Chinese quince are not particularly limited and may be appropriately selected depending on the intended purpose.

The extraction solvent of the Chinese quince is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Chinese quince is preferably used at room temperature or at a temperature equal to or lower than the boiling point of the solvent. Among them, the extraction is preferably performed with the hot water.

A method for purifying the Chinese quince extract is not particularly limited and may be appropriately selected depending on the intended purpose. The Chinese quince extract may be purified by, for example, a liquid-liquid distribution extraction, various chromatographies, and a membrane separation.

The concentration of the Chinese quince extract is not particularly limited and may be appropriately selected depending on the intended purpose, but is preferably 10 µg/mL to 400 µg/mL, more preferably 200 µg/mL to 400 µg/mL.

### <Psidium guava extract>

The Psidium guava (scientific name: Psidium guajava L.) is a scrub belonging to the genus Psidium of the family Myrtaceae. The Psidium guava is native to tropical America, and can be easily obtained from this region. The Psidium guava is also called as "banjiro" in Japan. The Psidium guava contains tannin, which is a polyphenolic compound, chlorophyll, folic acid, vitamins, minerals, proteins and polysaccharides, and has been widely utilized as health foods.

An extraction part of the Psidium guava is not particularly limited and may be appropriately selected depending on the intended purpose. Examples thereof include leaves, branches, trunks, barks, flowers, fruits and roots. Among them, preferred are fruits.

The extraction part of the Psidium guava can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Psidium guava extract can be easily obtained by using methods commonly used in extraction of plants. The Psidium guava extract is not particularly limited and may be appropriately selected depending on the intended purpose. For example, the Psidium guava extract may be in the form of diluted extract liquid, concentrated extract liquid or dried product of the extract.

A method for extracting the Psidium guava is not particularly limited and may be appropriately selected depending on the intended purpose. For example, the Psidium guava can be extracted as follows. Firstly, the extraction part of the Psidium guava, an extraction feed, is added to a processing tank containing an extraction solvent. Soluble components contained in the Psidium guava are allowed to dissolve in the extraction solvent while appropriately stirring, if necessary. Subsequently, extraction residue is removed by filtration to thereby obtain the Psidium guava extract.

Extraction conditions (extraction time and extraction temperature) of the Psidium guava and the amount of the extraction solvent of the Psidium guava are not particularly limited and may be appropriately selected depending on the intended purpose.

The extraction solvent of the Psidium guava is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Psidium guava is preferably used at room temperature or at a temperature equal to or lower than the boiling point of the solvent. Among them, the extraction is preferably performed with the hot water.

A method for purifying the Psidium guava extract is not particularly limited and may be appropriately selected depending on the intended purpose. The Psidium guava extract may be purified by, for example, a liquid-liquid distribution extraction, various chromatographies, and a membrane separation.

The concentration of the Psidium guava extract is not particularly limited and may be appropriately selected depending on the intended purpose, but is preferably 100 µg/mL to 800 µg/mL, more preferably 400 µg/mL to 800 µg/mL.

### <Long pepper extract>

The long pepper (scientific name: Piper longum) is an evergreen woody vine belonging to the genus Piper of the family Piperaceae. The long pepper can be easily obtained from Southeast Asia. Spikes of the long pepper are fleshy and cylindrical. Dried product of the spikes has been widely used as a spice.

An extraction part of the long pepper is not particularly limited and may be appropriately selected depending on the intended purpose. Examples thereof include spikes, leaves, stems, flowers and roots. Among them, preferred are spikes.

The extraction part of the long pepper can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The long pepper extract can be easily obtained by using methods commonly used in extraction of plants. The long pepper extract is not particularly limited and may be appropriately selected depending on the intended purpose. For example, the long pepper extract may be in the form of diluted extract liquid, concentrated extract liquid or dried product of the extract.

A method for extracting the long pepper is not particularly limited and may be appropriately selected depending on the intended purpose. For example, the long pepper can be extracted as follows. Firstly, the extraction part of the long pepper, an extraction feed, is added to a processing tank containing an extraction solvent. Soluble components contained in the long pepper are allowed to dissolve in the extraction solvent while appropriately stirring, if necessary. Subsequently, extraction residue is removed by filtration to thereby obtain the long pepper extract.

Extraction conditions (extraction time and extraction temperature) of the long pepper and the amount of the extraction solvent of the long pepper are not particularly limited and may be appropriately selected depending on the intended purpose.

The extraction solvent of the long pepper is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the long pepper is preferably used at room temperature or at a temperature equal to or lower than the boiling point of the solvent. Among them, the extraction is preferably performed with the hot water.

A method for purifying the long pepper extract is not particularly limited and may be appropriately selected depending on the intended purpose. The long pepper extract may be purified by, for example, a liquid-liquid distribution extraction, various chromatographies, and a membrane separation.

The concentration of the long pepper extract is not particularly limited and may be appropriately selected depending on the intended purpose, but is preferably 50 µg/mL to 400 µg/mL, more preferably 200 µg/mL to 400 µg/mL.

### <Quillaja extract>

The Quillaja (scientific name: Quillaja saponaria) is an evergreen small tree belonging to the genus Quillaja of the family Rosaceae. The Quillaja grows naturally in Chile, Bolivia and Peru of South America, and can be easily obtained from these regions. Barks of the Quillaja contain tannin and triterpene saponin. Saponin contained in the Quillaja (Quillaja saponin) has been known to have a more excellent surface activity and homogeneity than saponins obtained from other plants. Therefore, it has been widely utilized as an emulsifier for food, vitamin E, a solubilizer for perfume or a foaming agent for toothpaste or drink, or in toiletries and industrial fields such as photographic film.

An extraction part of the Quillaja is not particularly limited and may be appropriately selected depending on the intended purpose. Examples thereof include leaves, branches, trunks, barks, flowers, fruits and roots. Among them, preferred are barks.

The extraction part of the Quillaja can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Quillaja extract can be easily obtained by using methods commonly used in extraction of plants. The Quillaja extract is not particularly limited and may be appropriately selected depending on the intended purpose. For example, the Quillaja extract may be in the form of diluted extract liquid, concentrated extract liquid or dried product of the extract.

A method for extracting the Quillaja is not particularly limited and may be appropriately selected depending on the intended purpose. For example, the Quillaja can be extracted as follows. Firstly, the extraction part of the Quillaja, an extraction feed, is added to a processing tank containing an extraction solvent. Soluble components contained in the Quillaja are allowed to dissolve in the extraction solvent while appropriately stirring, if necessary. Subsequently, extraction residue is removed by filtration to thereby obtain the Quillaja extract.

Extraction conditions (extraction time and extraction temperature) of the Quillaja and the amount of the extraction solvent of the Quillaja are not particularly limited and may be appropriately selected depending on the intended purpose.

The extraction solvent of the Quillaja is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Quillaja is preferably used at room temperature or at a temperature equal to or lower than the boiling point of the solvent. Among them, the extraction is preferably performed with the hot water.

A method for purifying the Quillaja extract is not particularly limited and may be appropriately selected depending on the intended purpose. The Quillaja extract may be purified by, for example, a liquid-liquid distribution extraction, various chromatographies, and a membrane separation.

The concentration of the Quillaja extract is not particularly limited and may be appropriately selected depending on the intended purpose, but is preferably 6.25 µg/mL to 12.5 µg/mL.

### <Kouki extract>

The Kouki (scientific name: Engelhardtia chrysolepis) is an evergreen tree belonging to the genus Engelhardtia of the family Juglandaceae. The Kouki grows naturally in southern China, and can be easily obtained from this region. Leaves of the Kouki exhibit a weak sweet taste, so that they have been drunk for the purpose of defervescence, fat reducing or detoxification.

An extraction part of the Kouki is not particularly limited and may be appropriately selected depending on the intended purpose. Examples thereof include leaves, branches, trunks, barks, flowers, fruits and roots. Among them, preferred are leaves.

The extraction part of the Kouki can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Kouki extract can be easily obtained by using methods commonly used in extraction of plants. The Kouki extract is not particularly limited and may be appropriately selected depending on the intended purpose. For example, the Kouki extract may be in the form of diluted extract liquid, concentrated extract liquid or dried product of the extract.

A method for extracting the Kouki is not particularly limited and may be appropriately selected depending on the intended purpose. For example, the Kouki can be extracted as follows. Firstly, the extraction part of the Kouki, an extraction feed, is added to a processing tank containing an extraction solvent. Soluble components contained in the Kouki are allowed to dissolve in the extraction solvent while appropriately stirring, if necessary. Subsequently, extraction residue is removed by filtration to thereby obtain the Kouki extract.

Extraction conditions (extraction time and extraction temperature) of the Kouki and the amount of the extraction solvent of the Kouki are not particularly limited and may be appropriately selected depending on the intended purpose.

The extraction solvent of the Kouki is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Kouki is preferably used at room temperature or at a temperature equal to or lower than the boiling point of the solvent. Among them, the extraction is preferably performed with the hot water.

A method for purifying the Kouki extract is not particularly limited and may be appropriately selected depending on the intended purpose. The Kouki extract may be purified by, for example, a liquid-liquid distribution extraction, various chromatographies, and a membrane separation.

The concentration of the Kouki extract is not particularly limited and may be appropriately selected depending on the intended purpose, but is preferably 200 µg/mL to 400 µg/mL.

### <Ginkgo extract>

The ginkgo (scientific name: Ginkgo biloba) is a deciduous tree belonging to the genus Ginkgo of the family Ginkgoaceae. The ginkgo has been spread worldwide by grafting, and can be easily obtained from these regions. The ginkgo has stems 20 m to 30 m high. It is a species of gymnosperm. The ginkgo contains ginkgolide and flavonoid, which have been formulated into health food.

An extraction part of the ginkgo is not particularly limited and may be appropriately selected depending on the intended purpose. Examples thereof include leaves, branches, trunks, barks, flowers, fruits and roots. Among them, preferred are leaves.

The extraction part of the ginkgo can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The ginkgo extract can be easily obtained by using methods commonly used in extraction of plants. The ginkgo extract is not particularly limited and may be appropriately selected depending on the intended purpose. For example, the ginkgo extract may be in the form of diluted extract liquid, concentrated extract liquid or dried product of the extract.

A method for extracting the ginkgo is not particularly limited and may be appropriately selected depending on the intended purpose. For example, the ginkgo can be extracted as follows. Firstly, the extraction part of the ginkgo, an extraction feed, is added to a processing tank containing an extraction solvent. Soluble components contained in the ginkgo are allowed to dissolve in the extraction solvent while appropriately stirring, if necessary. Subsequently, extraction residue is removed by filtration to thereby obtain the ginkgo extract.

Extraction conditions (extraction time and extraction temperature) of the ginkgo and the amount of the extraction solvent of the ginkgo are not particularly limited and may be appropriately selected depending on the intended purpose.

The extraction solvent of the ginkgo is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the ginkgo is preferably used at room temperature or at a temperature equal to or lower than the boiling point of the solvent. Among them, the extraction is preferably performed with the hot water.

A method for purifying the ginkgo extract is not particularly limited and may be appropriately selected depending on the intended purpose. The ginkgo extract may be purified by, for example, a liquid-liquid distribution extraction, various chromatographies, and a membrane separation.

The concentration of the ginkgo extract is not particularly limited and may be appropriately selected depending on the intended purpose, but is preferably 100 µg/mL to 200 µg/mL.

### <Oyster extract>

The oyster is the general term for bivalves belonging to the family Ostreidae of the order Pterioida. The oyster used in the present invention was harvested in Hiroshima prefecture (Japan), but oysters harvested in other coastal regions all over the world may be used. The oyster contains good proteins, taurine, glycogen, zinc and minerals in a large amount. Therefore, the oyster has been widely used for raw material of Chinese herbal medicine from ancient times.

The oyster extract can be easily obtained by using methods commonly used in extraction of oysters. The oyster extract is not particularly limited and may be appropriately selected depending on the intended purpose. For example, the oyster extract may be in the form of diluted extract liquid, concentrated extract liquid or dried product of the extract.

A method for extracting the oyster is not particularly limited and may be appropriately selected depending on the intended purpose. Examples thereof include (1) a method in which refrigerated raw oysters and frozen oysters are charged into an extraction container, and then sequentially subjected to a thermal extraction using hot water of 80°C to 100°C, a clarifying filtration, and a vacuum concentration or (2) a method in which hot water extraction residue of oysters is charged into a reaction tank equipped with a mixing impeller, treated with protease at 30°C to 50°C, inactivated by heating at 90°C, and then sequentially subjected to a clarifying filtration and a vacuum concentration.

The extraction solvent of the oyster is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the oyster is preferably used at room temperature or at a temperature equal to or lower than the boiling point of the solvent. Among them, the extraction is preferably performed with the hot water.

The amount of the extraction solvent of the oyster is not particularly limited and may be appropriately selected depending on the intended purpose.

A method for purifying the oyster extract is not particularly limited and may be appropriately selected depending on the intended purpose. The oyster extract may be purified by, for example, a liquid-liquid distribution extraction, various chromatographies, and a membrane separation.

The concentration of the oyster extract is not particularly limited and may be appropriately selected depending on the intended purpose, but is preferably 200 µg/mL to 400 µg/mL.

### <Turmeric extract>

The turmeric (scientific name: Curcuma longa) is a perennial herb belonging to the genus Curcuma of the family Zingiberaceae. The turmeric is distributed in tropical Asia, and cultivated in India, Okinawa, southeastern China and Southeast Asia. The turmeric can be easily obtained from these regions. Tuberous roots of the turmeric contains a yellow dye curcumine, which has been known to have an antioxidative effect, a liver function improving effect and a prostatic hypertrophy inhibitory effect. In addition, the turmeric also contains an essential oil component which is called as sesquiterpene. Fleshy rhizomes of the turmeric have been utilized for medicinal purposes, and as a major spice and a yellow dye in curry powder.

An extraction part of the turmeric is not particularly limited and may be appropriately selected depending on the intended purpose. Examples thereof include leaves, stems, flowers, fruits, rhizomes and roots. Among them, preferred are rhizomes.

The extraction part of the turmeric can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The turmeric extract can be easily obtained by using methods commonly used in extraction of plants. The turmeric extract is not particularly limited and may be appropriately selected depending on the intended purpose. For example, the turmeric extract may be in the form of diluted extract liquid, concentrated extract liquid or dried product of the extract.

A method for extracting the turmeric is not particularly limited and may be appropriately selected depending on the intended purpose. For example, the turmeric can be extracted as follows. Firstly, the extraction part of the turmeric, an extraction feed, is added to a processing tank containing an extraction solvent. Soluble components contained in the turmeric are allowed to dissolve in the extraction solvent while appropriately stirring, if necessary. Subsequently, extraction residue is removed by filtration to thereby obtain the turmeric extract.

Extraction conditions (extraction time and extraction temperature) of the turmeric and the amount of the extraction solvent of the turmeric are not particularly limited and may be appropriately selected depending on the intended purpose.

The extraction solvent of the turmeric is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the turmeric is preferably used at room temperature or at a temperature equal to or lower than the boiling point of the solvent. Among them, the extraction is preferably performed with the hot water.

A method for purifying the turmeric extract is not particularly limited and may be appropriately selected depending on the intended purpose. The turmeric extract may be purified by, for example, a liquid-liquid distribution extraction, various chromatographies, and a membrane separation.

The concentration of the turmeric extract is not particularly limited and may be appropriately selected depending on the intended purpose, but is preferably 25 µg/mL to 40 µg/mL.

### <Chrysanthemum extract>

The chrysanthemum (scientific name: Chrysanthemum morifolium syn.) is a short-day plant belonging to the genus Chrysanthemum of the family Asteraceae. The chrysanthemum grows naturally and can be easily obtained in Japan. Flowers of the chrysanthemum have been utilized for medicinal purposes in China from ancient times. In Japan, the chrysanthemum has been eaten or drunk as tea or liquor. The chrysanthemum has been believed to be effective for dizziness, headache and eyestrain.

An extraction part of the chrysanthemum is not particularly limited and may be appropriately selected depending on the intended purpose. Examples thereof include leaves, stems, flowers, fruits and roots. Among them, preferred are flowers.

The extraction part of the chrysanthemum can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The chrysanthemum extract can be easily obtained by using methods commonly used in extraction of plants. The chrysanthemum extract is not particularly limited and may be appropriately selected depending on the intended purpose. For example, the chrysanthemum extract may be in the form of diluted extract liquid, concentrated extract liquid or dried product of the extract.

A method for extracting the chrysanthemum is not particularly limited and may be appropriately selected depending on the intended purpose. For example, the chrysanthemum can be extracted as follows. Firstly, the extraction part of the chrysanthemum, an extraction feed, is added to a processing tank containing an extraction solvent. Soluble components contained in the chrysanthemum are allowed to dissolve in the extraction solvent while appropriately stirring, if necessary. Subsequently, extraction residue is removed by filtration to thereby obtain the chrysanthemum extract.

Extraction conditions (extraction time and extraction temperature) of the chrysanthemum and the amount of the extraction solvent of the chrysanthemum are not particularly limited and may be appropriately selected depending on the intended purpose.

The extraction solvent of the chrysanthemum is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the chrysanthemum is preferably used at room temperature or at a temperature equal to or lower than the boiling point of the solvent. Among them, the extraction is preferably performed with the hot water.

A method for purifying the chrysanthemum extract is not particularly limited and may be appropriately selected depending on the intended purpose. The chrysanthemum extract may be purified by, for example, a liquid-liquid distribution extraction, various chromatographies, and a membrane separation.

The concentration of the chrysanthemum extract is not particularly limited and may be appropriately selected depending on the intended purpose, but is preferably 200 µg/mL to 400 µg/mL.

### <Jujube extract>

The jujube (scientific name: Ziziphus jujuba) is a deciduous tree belonging to the genus Ziziphus of the family Rhamnaceae. The jujube is native to north Africa and west Europe, and can be easily obtained from these regions. Fruits of the jujube have been utilized for medicinal purposes from ancient times. The jujube also contains vitamin C.

An extraction part of the jujube is not particularly limited and may be appropriately selected depending on the intended purpose. Examples thereof include leaves, branches, trunks, barks, flowers, fruits and roots. Among them, preferred are fruits.

The extraction part of the jujube can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The jujube extract can be easily obtained by using methods commonly used in extraction of plants. The jujube extract is not particularly limited and may be appropriately selected depending on the intended purpose. For example, the jujube extract may be in the form of diluted extract liquid, concentrated extract liquid or dried product of the extract.

A method for extracting the jujube is not particularly limited and may be appropriately selected depending on the intended purpose. For example, the jujube can be extracted as follows. Firstly, the extraction part of the jujube, an extraction feed, is added to a processing tank containing an extraction solvent. Soluble components contained in the jujube are allowed to dissolve in the extraction solvent while appropriately stirring, if necessary. Subsequently, extraction residue is removed by filtration to thereby obtain the jujube extract.

Extraction conditions (extraction time and extraction temperature) of the jujube and the amount of the extraction solvent of the jujube are not particularly limited and may be appropriately selected depending on the intended purpose.

The extraction solvent of the jujube is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the jujube is preferably used at room temperature or at a temperature equal to or lower than the boiling point of the solvent. Among them, the extraction is preferably performed with the hot water.

A method for purifying the jujube extract is not particularly limited and may be appropriately selected depending on the intended purpose. The jujube extract may be purified by, for example, a liquid-liquid distribution extraction, various chromatographies, and a membrane separation.

The concentration of the jujube extract is not particularly limited and may be appropriately selected depending on the intended purpose, but is preferably 200 µg/mL to 400 µg/mL.

### <Chinese wolfberry extract>

The Chinese wolfberry (scientific name: Lycium chinense) is a deciduous shrub belonging to the genus Lycium of the family Solanaceae. The jujube is distributed in Japan, Korea, China and Taiwan, and can be easily obtained from these regions. Fruits of the Chinese wolfberry have been drunk as Chinese wolfberry liquor made by preserving them in liquor. Also, they have been eaten as fresh fruits or dried fruits, and as ingredients of rice gruel in Chinese food therapy. Soft young leaves of the Chinese wolfberry have been widely utilized for food.

An extraction part of the Chinese wolfberry is not particularly limited and may be appropriately selected depending on the intended purpose. Examples thereof include leaves, branches, trunks, barks, flowers, fruits and roots. Among them, preferred are fruits.

The extraction part of the Chinese wolfberry can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Chinese wolfberry extract can be easily obtained by using methods commonly used in extraction of plants. The Chinese wolfberry extract is not particularly limited and may be appropriately selected depending on the intended purpose. For example, the Chinese wolfberry extract may be in the form of diluted extract liquid, concentrated extract liquid or dried product of the extract.

A method for extracting the Chinese wolfberry is not particularly limited and may be appropriately selected depending on the intended purpose. For example, the Chinese wolfberry can be extracted as follows. Firstly, the extraction part of the Chinese wolfberry, an extraction feed, is added to a processing tank containing an extraction solvent. Soluble components contained in the Chinese wolfberry are allowed to dissolve in the extraction solvent while appropriately stirring, if necessary. Subsequently, extraction residue is removed by filtration to thereby obtain the Chinese wolfberry extract.

Extraction conditions (extraction time and extraction temperature) of the Chinese wolfberry and the amount of the extraction solvent of the Chinese wolfberry are not particularly limited and may be appropriately selected depending on the intended purpose.

The extraction solvent of the Chinese wolfberry is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Chinese wolfberry is preferably used at room temperature or at a temperature equal to or lower than the boiling point of the solvent. Among them, the extraction is preferably performed with the hot water.

A method for purifying the Chinese wolfberry extract is not particularly limited and may be appropriately selected depending on the intended purpose. The Chinese wolfberry extract may be purified by, for example, a liquid-liquid distribution extraction, various chromatographies, and a membrane separation.

The concentration of the Chinese wolfberry extract is not particularly limited and may be appropriately selected depending on the intended purpose, but is preferably 100 µg/mL to 400 µg/mL.

### <Chamomile extract>

The chamomile (scientific name: Matricaria chamomilla) is a cold-resistant annual herb belonging to the genus Matricaria of the family Asteraceae. The chamomile is native to Europe and west Asia, and can be easily obtained from these regions. Leaves of the chamomile are pinnately compound leaves. Flowers of the chamomile have a diameter of about 3 cm, have yellow tubulous flowers at the center and white ligulate flowers, and have a unique strong apple-like aroma. The chamomile has been known to be a medicinal herb as a stomachic, a diaphoretic, an anti-inflammatory agent or a drug for gynecopathy.

An extraction part of the chamomile is not particularly limited and may be appropriately selected depending on the intended purpose. Examples thereof include leaves, stems, flowers, fruits and roots. Among them, preferred are flowers.

The extraction part of the chamomile can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The chamomile extract can be easily obtained by using methods commonly used in extraction of plants. The chamomile extract is not particularly limited and may be appropriately selected depending on the intended purpose. For example, the chamomile extract may be in the form of diluted extract liquid, concentrated extract liquid or dried product of the extract.

A method for extracting the chamomile is not particularly limited and may be appropriately selected depending on the intended purpose. For example, the chamomile can be extracted as follows. Firstly, the extraction part of the chamomile, an extraction feed, is added to a processing tank containing an extraction solvent. Soluble components contained in the chamomile are allowed to dissolve in the extraction solvent while appropriately stirring, if necessary. Subsequently, extraction residue is removed by filtration to thereby obtain the chamomile extract.

Extraction conditions (extraction time and extraction temperature) of the chamomile and the amount of the extraction solvent of the chamomile are not particularly limited and may be appropriately selected depending on the intended purpose.

The extraction solvent of the chamomile is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the chamomile is preferably used at room temperature or at a temperature equal to or lower than the boiling point of the solvent. Among them, the extraction is preferably performed with the hot water.

A method for purifying the chamomile extract is not particularly limited and may be appropriately selected depending on the intended purpose. The chamomile extract may be purified by, for example, a liquid-liquid distribution extraction, various chromatographies, and a membrane separation.

The concentration of the chamomile extract is not particularly limited and may be appropriately selected depending on the intended purpose, but is preferably 200 µg/mL to 400 µg/mL.

### <Butcher's Broom extract>

The Butcher's Broom (scientific name: Ruscus aculeatus, Japanese name: Nagiikada) is a perennial evergreen shrub belonging to the genus Ruscus of the family Asparagaceae. The Butcher's Broom is native to Europe, and can be easily obtained from this region. The Butcher's Broom is a dioecious plant. The Butcher's Broom produces flowers from spring to summer and produces red fruits in winter. Leaves of the Butcher's Broom are degenerated. Stems are flattened like a leaf at ends and have a sharp spine at a tip.

An extraction part of the Butcher's Broom is not particularly limited and may be appropriately selected depending on the intended purpose. Examples thereof include leaves, stems, flowers, fruits, roots and rhizomes. Among them, preferred are rhizomes.

The extraction part of the Butcher's Broom can be prepared as follows. Each of the extraction part may be dried and directly used in a solvent extraction process, or it may be dried, crushed by a crusher, and then subjected to the solvent extraction process. The extraction part may be dried by sun drying or by using a commonly used drier.

The Butcher's Broom extract can be easily obtained by using methods commonly used in extraction of plants. The Butcher's Broom extract is not particularly limited and may be appropriately selected depending on the intended purpose. For example, the Butcher's Broom extract may be in the form of diluted extract liquid, concentrated extract liquid or dried product of the extract.

A method for extracting the Butcher's Broom is not particularly limited and may be appropriately selected depending on the intended purpose. For example, the Butcher's Broom can be extracted as follows. Firstly, the extraction part of the Butcher's Broom, an extraction feed, is added to a processing tank containing an extraction solvent. Soluble components contained in the Butcher's Broom are allowed to dissolve in the extraction solvent while appropriately stirring, if necessary. Subsequently, extraction residue is removed by filtration to thereby obtain the Butcher's Broom extract.

Extraction conditions (extraction time and extraction temperature) of the Butcher's Broom and the amount of the extraction solvent of the Butcher's Broom are not particularly limited and may be appropriately selected depending on the intended purpose.

The extraction solvent of the Butcher's Broom is not particularly limited and may be appropriately selected depending on the intended purpose. Examples thereof include water, hydrophilic solvents or mixed solvents thereof. The water is not particularly limited and may be appropriately selected depending on the intended purpose. Examples thereof include pure water, tap water, well water, mineral spring water, mineral water, hot spring water, spring water, fresh water, purified water, hot water, ion-exchanged water, physiological saline, phosphate buffer and phosphate buffered saline. The hydrophilic solvents are not particularly limited and may be appropriately selected depending on the intended purpose. Example thereof include lower alcohols having 1 carbon atom to 5 carbon atoms such as methanol, ethanol, propyl alcohol and isopropyl alcohol; lower aliphatic ketones such as acetone and methyl ethyl ketone; and polyhydric alcohols having 2 carbon atoms to 5 carbon atoms such as 1,3-butylene glycol, propylene glycol and glycerol. The mixed solvents are not particularly limited and may be appropriately selected depending on the intended purpose. In the case of using the lower alcohols, 1 part by mass to 90 parts by mass of the lower alcohols are preferably added relative to 10 parts by mass of the water. In the case of using the lower aliphatic ketones, 1 part by mass to 40 parts by mass of the lower aliphatic ketones are preferably added relative to 10 parts by mass of the water. In the case of using the polyhydric alcohols, 1 part by mass to 90 parts by mass of the polyhydric alcohols are preferably added relative to 10 parts by mass of the water. Notably, the extraction solvent of the Butcher's Broom is preferably used at room temperature or at a temperature equal to or lower than the boiling point of the solvent. Among them, the extraction is preferably performed with the hot water.

A method for purifying the Butcher's Broom extract is not particularly limited and may be appropriately selected depending on the intended purpose. The Butcher's Broom extract may be purified by, for example, a liquid-liquid distribution extraction, various chromatographies, and a membrane separation.

The concentration of the Butcher's Broom extract is not particularly limited and may be appropriately selected depending on the intended purpose, but is preferably 50 µg/mL to 200 µg/mL, more preferably 100 µg/mL to 200 µg/mL.

### <Other ingredients>

The other ingredients are not particularly limited and may be appropriately selected depending on the intended purpose. Example thereof include excipients, desiccants, preservatives, nutrient supplements, thickeners, emulsifiers, antioxidants, sweeteners, acidulants, seasonings, colorants, flavoring agents, whitening agents, moisturizers, oily ingredients, ultraviolet absorbers, surfactants, thickening agents, alcohols, powder ingredients, coloring agents, aqueous ingredients, water or skin nutrients.

Also, the other ingredients are not particularly limited and may be appropriately selected depending on the intended purpose. Specific examples thereof include hyaluronic acid, hyaluronic acid hydrolysates, collagen, collagen hydrolysates, ascorbic acid, ascorbic acid derivatives, ascorbic acid glycosides, Coenzyme Q10, propolis, royal jelly, royal jelly proteolysates, fucoidan, aloe powder, aloe extract, blueberry powder, blueberry extract, isoflavone, noni powder, noni extract, garlic powder, garlic extract, turmeric powder, chitosan, glucosamine, chlorella powder, chlorella extract, carnitine, maca powder, maca extract, black currant powder, black currant extract, Sparassis crispa powder, Sparassis crispa extract, and other powder, or extracts of plants which are effective for beauty, or any combination thereof.

### <Application>

The Tie2 activator, the vascular endothelial growth factor (VEGF) inhibitor, the angiogenesis inhibitor, the vascular maturing agent, the vascular normalizing agent and the vascular stabilizing agent, and the pharmaceutical composition of the present invention have an excellent Tie2 activating effect, vascular endothelial growth factor (VEGF) inhibitory effect, angiogenesis inhibitory effect, vascular maturing effect, vascular normalizing effect, and vascular stabilizing effect, so that they can be suitably used as pharmaceuticals for diseases with vascular lesions such as tumors, chronic rheumatoid arthritis, diabetic retinopathy, hyperlipemia or hypertension and allergic diseases such as atopic dermatitis or hay fever; and as safe preventive agents for the above-described diseases. The amount, usage, and dosage form thereof may be appropriately selected depending on the intended purpose.

The Tie2 activator, the vascular endothelial growth factor (VEGF) inhibitor, the angiogenesis inhibitor, the vascular maturing agent, the vascular normalizing agent, and the vascular stabilizing agent of the present invention have been verified to not be digested in a digestive tract. Therefore, they can be suitably used as food and drink such as cosmetic food and drink or health food and drink. The amount, usage, and dosage form thereof may be appropriately selected depending on the intended purpose.

The amount may be appropriately adjusted depending on bioactivity of the extracts, but is preferably 0.0001% by mass to 20% by mass, more preferably 0.0001% by mass to 10% by mass on a purified product basis of the hawthorn extract, the starfruit extract, the shellflower extract, the lotus extract, the rooibos extract, the Indian date extract, the Chinese quince extract, the Psidium guava extract, the long pepper extract, the Quillaja extract, the Kouki extract, the ginkgo extract, the oyster extract, the turmeric extract, the chrysanthemum extract, the jujube extract, the Chinese wolfberry extract, the chamomile extract, or the Butcher's Broom extract, or any combination thereof.

The usage is not particularly limited and may be appropriately selected depending on the intended purpose. Examples thereof include an oral dosage, a parenteral dosage and an external dosage.

The dosage form is not particularly limited and may be appropriately selected depending on the intended purpose. Examples thereof include oral dosage forms such as tablets, powders, capsules, granules, extracts and syrups; parenteral dosage forms such as injections, intravenous drips and suppositories; and external dosage forms such as ointments, creams, milky lotions, lotions, facial packs, bath additives and hair cosmetics.

### Examples

Examples of the present invention now will be explained, but the present invention is not limited thereto.

### [Test Example 1: Tie2 activating effect (Western blot) test]

### (Example 1: hawthorn extract)

The pharmacological effect of the hawthorn extract was evaluated by detecting the amount of phosphorylated Tie2 protein. The phosphorylated Tie2 protein was quantified by means of an immunochemical method including SDS-PAGE and Western blot. The method was performed as follows.

At first, 2×10⁵ of human umbilical vein endothelial cells (HUVECs) were inoculated in a 6-well culture plate, and then cultured in HUMEDIA EG2 medium (product of KURABO INDUSTRIES LTD.) for 12 hours within a CO₂ incubator at 37°C. Thereafter, the medium was removed. The cells were washed with PBS and cultured in RPMI-1640 medium (product by SIGMA, R-8755) for 2 hours. The hawthorn extract (fruits) (HAWTHORN EXTRACT POWDER MF, product of MARUZEN PHARMACEUTICALS CO., LTD.) was dissolved in DMSO so as to have a final concentration of 100 µg/mL, followed by adding to the medium, incubating for 10 minutes, cooling the cells on ice and washing with cold PBS. The cells were then subjected to an ultrasonic grinding in RIPA buffer containing protease inhibitors (Leupeptin, Aprotinin, Pepstatin, PMSF and Na₃VO₄). Sample buffer (0.2 M Tris-HCl (pH 6.8), 4% by mass SDS, 20% by mass glycerol, 5 mM EDTA and 0.01% by mass BPB) was then added thereto, which was referred to as Sample 1. Sample 1 was subjected to SDS-PAGE (7.5% by mass polyacrylamide gel, 12 wells, NPU-7.5L, product of ATTO CORPORATION) under the following conditions.

Gel: 7.5% by mass acrylamide gel (NPU-7.5L, product of ATTO CORPORATION)

Electrophoresis conditions: 40 mA (75 min, 2 gel sheets)

Phospho-Tie2 antibody: #4221 (product of Cell Sigma).

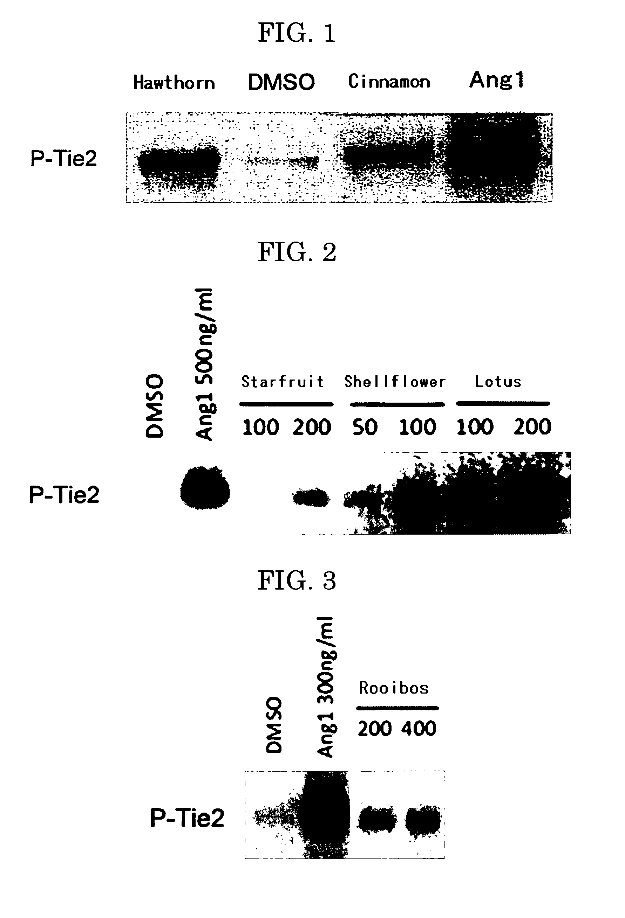

Next, the gel was transferred onto a PVDF membrane and subjected to Western blotting (20 V, 4°C, overnight). After blocking (1 hour) with 5% by mass skim milk/TBST, phosphorylated Tie2 (Cell Signaling, #4221) was added thereto followed by allowing to stand 1 hour at room temperature. Then, goat anti-rabbit Ig's HRP (BIOSOURCE ALI3404) was added thereto followed by allowing to stand for 1 hour at room temperature. Protein bands were detected by chemiluminescence detection (ECL). Results are shown in FIG. 1.

### (Example 2: starfruit extract)

The pharmacological effect of the starfruit extract was evaluated by detecting the amount of phosphorylated Tie2 protein. The phosphorylated Tie2 protein was quantified by means of an immunochemical method including SDS-PAGE and Western blot. The method was performed as follows.

Tie2 phosphorylation analysis was performed using mouse pro-B cells (Ba/F3) overexpressing human Tie2 (Ba/F3-human Tie2). The mouse pro-B cells were stimulated by adding the starfruit extracts (STARFRUIT LEAF EXTRACT POWDER MF, product of MARUZEN PHARMACEUTICALS CO., LTD.) (concentration unit: µg/mL) having predetermined concentrations (i.e., concentrations described in FIG. 2, concentration unit: µg/mL) to normal culture media (10% FBS RPMI 1640 (product of SIGMA, R-8755) + 1 pg/mL mouse IL-3) at 37°C. After 15 min of stimulation, the cells were washed with cold PBS. Cell extracts were collected in RIPA lysis buffers (50 mM Tris-HCl (pH7.5), 150 mM NaCl, 1% by mass of NP-40, 0.5% by mass of sodium deoxycholate and 0.1% by mass of SDS). The cell extracts were subjected to electrophoresis on 7.5% by mass of SDS gel (NPU-7.5L, product of ATTO CORPORATION) and transferred onto a nylon membrane. The transferred nylon membrane was blocked with 5% by mass of skim milk/TBST for 60 min to inhibit non-specific protein bindings followed by sequentially blotting with anti-Ti2e antibodies (Ab33: product of Upstate Biotechnology, Inc.), anti-phosphorylated Tie2 antibodies (Tyr992: product of Cell Signaling Technology, Inc.) and HRP-labeled secondary antibodies. The resultant reaction products were visualized with an electrochemiluminescence (ECL) solution as protein bands and photographed. Results are shown in FIG. 2.

### (Example 3: shellflower extract)

The tests were performed in the same manner as in Example 2, except that the starfruit extract was changed to the shellflower extract (SHELLFLOWER LEAF DRY EXTRACT F, product of MARUZEN PHARMACEUTICALS CO., LTD.) and concentrations described in FIG. 2 (concentration unit: µg/mL) were used. Results are shown in FIG. 2.

### (Example 4: lotus extract)

The tests were performed in the same manner as in Example 2, except that the starfruit extract was changed to the lotus extract (LOTUS GERM EXTRACT POWDER MF, product of MARUZEN PHARMACEUTICALS CO., LTD.) and concentrations described in FIG. 2 (concentration unit: µg/mL) were used. Results are shown in FIG. 2.

### (Example 5: rooibos extract)

The tests were performed in the same manner as in Example 2, except that the starfruit extract was changed to the rooibos extract (ROOIBOS TEA DRY EXTRACT F, product of MARUZEN PHARMACEUTICALS CO., LTD.) and concentrations described in FIG. 3 (concentration unit: µg/mL) were used. Results are shown in FIG. 3.

### (Example 6: Indian date extract)

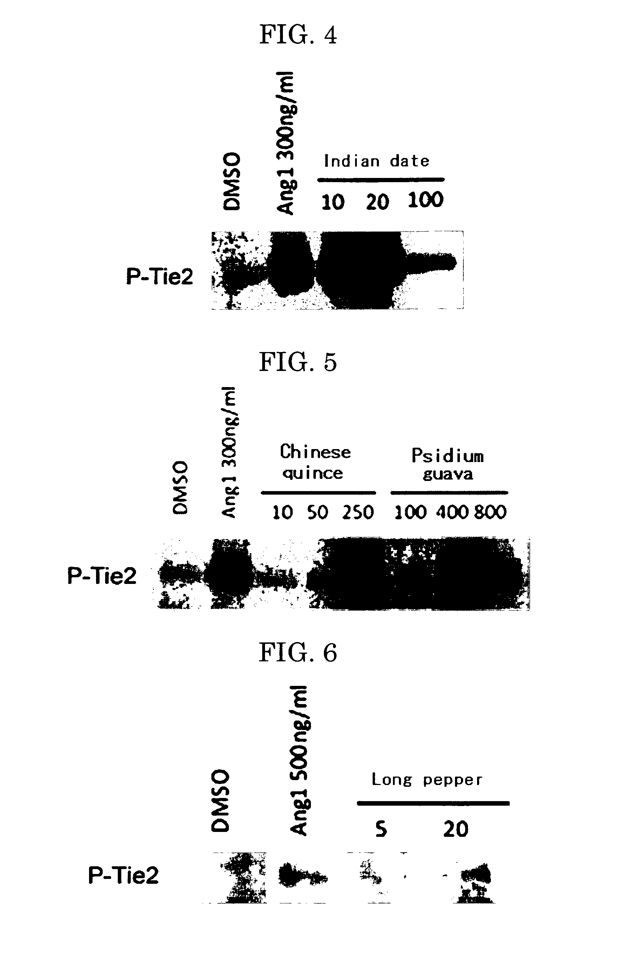

The tests were performed in the same manner as in Example 2, except that the starfruit extract was changed to the Indian date extract (INDIAN DATE EXTRACT POWDER MF, product of MARUZEN PHARMACEUTICALS CO., LTD.) and concentrations described in FIG. 4 (concentration unit: µg/mL) were used. Results are shown in FIG. 4.

### (Example 7: Chinese quince extract)

The tests were performed in the same manner as in Example 2, except that the starfruit extract was changed to the Chinese quince extract (CHINESE QUINCE EXTRACT POWDER MF, product of MARUZEN PHARMACEUTICALS CO., LTD.) and concentrations described in FIG. 5 (concentration unit: µg/mL) were used. Results are shown in FIG. 5.

### (Example 8: Psidium guava extract)

The tests were performed in the same manner as in Example 2, except that the starfruit extract was changed to the Psidium guava extract (PSIDIUM GUAVA DRY EXTRACT F, product of MARUZEN PHARMACEUTICALS CO., LTD.) and concentrations described in FIG. 5 (concentration unit: µg/mL) were used. Results are shown in FIG. 5.

### (Example 9: long pepper extract)

The pharmacological effect of the long pepper extract was evaluated by detecting the amount of phosphorylated Tie2 protein. The phosphorylated Tie2 protein was quantified by means of an immunochemical method including SDS-PAGE and Western blot. The method was performed as follows.

Tie2 phosphorylation analysis was performed using Human umbilical vein endothelial cells (HUVECs). The HUVECs were stimulated with Ang1 and test samples as follows. At first, the HUVECs were cultured in RPMI-1640 medium containing 1% FBS for 3 hours. Then, the test samples having various concentrations were added thereto. After 20 min, the cells were washed with cold PBS. Cell extracts were collected in RIPA lysis buffers (50mM Tris-HCl (pH7.5), 150mM NaCl, 1% by mass of NP-40, 0.5% by mass of sodium deoxycholate and 0.1% by mass of SDS). The cell extracts were subjected to electrophoresis on 7.5% by mass of SDS gel and transferred onto a nylon membrane. The transferred nylon membrane was blocked with 5% by mass of skim milk/TBST for 60 min to inhibit non-specific protein bindings, followed by sequentially blotting with anti-Ti2e antibodies (Ab33: product of Upstate Biotechnology, Inc.), anti-phosphorylated Tie2 antibodies (Tyr992: product of Cell Signaling Technology, Inc.) and HRP-labeled secondary antibodies. The resultant reaction products were visualized with an electrochemiluminescence (ECL) solution as protein bands and photographed. The concentrations of the bands were measured using Malti Guage-Image software of Las 3000-mini to thereby calculate as p-Tie2 (each test sample)/p-Tie2 (control). Results are shown in FIG. 6.

### (Comparative Example 1: cinnamon extract)

The test was performed in the same manner as in Example 1, except that the hawthorn extract was changed to a cinnamon extract (CINNAMON EXTRACT POWDER MF, product of MARUZEN PHARMACEUTICALS CO., LTD.) Notably, the cinnamon extract (CINNAMON EXTRACT POWDER MF, product of MARUZEN PHARMACEUTICALS CO., LTD.) was prepared to have a final concentration of 100 µg/mL in DMSO. Results are shown in FIG. 1.

### (Referential Example 1: positive control)

The test was performed in the same manner as in Example 1, except that the hawthorn extract was changed to Angiopoietin-1 (product of R&D Systems, Inc.) as a positive control. Notably, the Angiopoietin-1 (product of R&D Systems, Inc.) was prepared to have a final concentration of 300 ng/mL in DMSO. Results are shown in FIG. 1.

### (Referential Example 2: negative control)

The test was performed in the same manner as in Example 1, except that the hawthorn extract was changed to dimethyl sulfoxide (DMSO) as a negative control. Results are shown in FIG. 1.

### (Referential Example 3: positive control)

The tests were performed in the same manner as in Examples 2 to 9, except that the extracts were changed to Angiopoietin-1 (product of R&D Systems, Inc.) as a positive control and concentrations described in FIGs (concentration unit: µg/mL) were used. Results are shown in each FIG.

### (Referential Example 4: negative control)

The tests were performed in the same manner as in Examples 2 to 9, except that each of the extracts was changed to dimethyl sulfoxide (DMSO) as a negative control. Results are shown in each FIG.

### [Test Example 1: test result]

The test results of the Tie2 activating effect (Western blot) now will be explained.

FIGs. 1 to 6 show bands of phosphorylated Tie2 protein in each sample detected by Western blot. The density of the detected band is in proportion to the amount of phosphorylated Tie2 protein. The higher the density is, the more the amount of phosphorylated Tie2 protein is. It was verified from FIG. 1 that the hawthorn extract used in Example resulted in significant phosphorylation of Tie2 similar to Angiopoietin-1. The cinnamon which had been reported to phosphorylate Tie2 was also verified to induce phosphorylation of Tie2. However, the hawthorn extract had higher ability to induce phosphorylation of Tie2 than that of the cinnamon. Meanwhile, when using DMSO as a negative control, substantially no Tie2 was phosphorylated. Based on Western blot using Tie2 antibodies (Santa Cruz, sc-324), Tie2 was found to be expressed in the same amounts in all samples.

In addition, it was verified from FIGs. 4 to 6 that the Psidium guava extract, the Indian date extract, and the long pepper extract induced phosphorylation of Tie2 on similar level with Angiopoietin-1 which is biologically active substance. Among them, the Indian date extract was found to have an activating potency close to that of Angiopoietin-1 even when used at low concentration. It was verified from FIGs. 2, 3 and 5 that the starfruit extract, the shellflower extract, the rooibos extract, the lotus extract, and the Chinese quince extract also induced phosphorylation of Tie2, but their activating potencies were low.

Notably, Western blots were performed using HUVECs for the Psidium guava extract, the Indian date extract, the Chinese quince extract, the starfruit extract, the shellflower extract, the rooibos extract and the lotus extract, similar results to the above results were obtained.

Thus, the extracts used in Examples were found to induce activation of Tie2.

### [Test Example 2: Tie2 activating effect (immunoassay) test]

### (Example 10: hawthorn extract)

The pharmacological effect of the hawthorn extract was evaluated by detecting the amount of phosphorylated Tie2 protein. The phosphorylated Tie2 protein was quantified by means of an immunochemical method including an immunoassay. The method was performed as follows.

At first, normal human umbilical vein endothelial cells (HUVECs) were cultured to confluent, inoculated into a 96-well plate at 2.0×10⁴ cells/0.1 mL/well, and then cultured over night in a low-serum growth medium for vascular endothelial cells (HUMEDIA-EG2, product of KURABO INDUSTRIES LTD.) Next, the low-serum growth medium was replaced by 0.1 mL of a basal medium for vascular endothelial cells (HUMEDIA-EB2, product of KURABO INDUSTRIES LTD.) at 3 hours before cell stimulation (addition of test samples), and the post-cultured HUVECs were again cultured therein. Then, 0.1 mL of test samples, the hawthorn extract (leaves) (HAWTHORN LEAF EXTRACT POWDER, product of MARUZEN PHARMACEUTICALS CO., LTD.) which had been diluted with the HUMEDIA-EB2 to concentrations described in Table 1-1, were added into the wells, following by incubation for 10 min. After completion of the incubation, the amount of phosphorylated Tie2 and the total amount of Tie2 in the cells were measured by the immunoassay kit (HUMAN PHOSPHO-TIE2 (Y992) IMMUNOASSAY, product of R&D Systems) according to a manufacturer's protocol to thereby calculate the ratio of the amount of phosphorylated Tie2 to the total amount of Tie2.

Similarly, the ratio of the amount of phosphorylated Tie2 to the total amount of Tie2 was calculated in the case of using dimethyl sulfoxide (DMSO) as a negative control.

The activation rate of Tie2 was calculated according to the following Equation (1) to thereby evaluate a phosphorylating effect. Results are shown in Table 1-1.

Activation rate of Tie2 (%) = [{(Measured amount of phosphorylated Tie2 with addition of test sample)/(Measured amount of total Tie2 with addition of test sample)}/{(Measured amount of phosphorylated Tie2 with addition of negative control)/(Measured amount of total Tie2 with addition of negative control)}]x 100···Equation (1)

### (Example 11: starfruit extract)

The tests were performed in the same manner as in Example 10, except that the hawthorn extract was changed to the starfruit extract (STARFRUIT LEAF EXTRACT POWDER MF, product of MARUZEN PHARMACEUTICALS CO., LTD.) and concentrations described in Table 1-1 were used. Results are shown in Table 1-1.

### (Example 12: shellflower extract)

The tests were performed in the same manner as in Example 10, except that the hawthorn extract was changed to the shellflower extract (SHELLFLOWER LEAF DRY EXTRACT F, product of MARUZEN PHARMACEUTICALS CO., LTD.) and concentrations described in Table 1-1 were used. Results are shown in Table 1-1.

### (Example 13: lotus extract)

The tests were performed in the same manner as in Example 10, except that the hawthorn extract was changed to the lotus extract (LOTUS GERM EXTRACT POWDER MF, product of MARUZEN PHARMACEUTICALS CO., LTD.) and concentrations described in Table 1-1 were used. Results are shown in Table 1-1.

### (Example 14: rooibos extract)

The tests were performed in the same manner as in Example 10, except that the hawthorn extract was changed to the rooibos extract (ROOIBOS TEA DRY EXTRACT F, product of MARUZEN PHARMACEUTICALS CO., LTD.) and concentrations described in Table 1-1 were used. Results are shown in Table 1-1.

### (Example 15: Indian date extract)

The tests were performed in the same manner as in Example 10, except that the hawthorn extract was changed to the Indian date extract (INDIAN DATE EXTRACT POWDER MF, product of MARUZEN PHARMACEUTICALS CO., LTD.) and concentrations described in Table 1-1 were used. Results are shown in Table 1-1.

### (Example 16: Chinese quince extract)

The tests were performed in the same manner as in Example 10, except that the hawthorn extract was changed to the Chinese quince extract (CHINESE QUINCE EXTRACT POWDER MF, product of MARUZEN PHARMACEUTICALS CO., LTD.) and concentrations described in Table 1-1 were used. Results are shown in Table 1-1.

### (Example 17: Psidium guava extract)

The tests were performed in the same manner as in Example 10, except that the hawthorn extract was changed to the Psidium guava extract (PSIDIUM GUAVA DRY EXTRACT F, product of MARUZEN PHARMACEUTICALS CO., LTD.) and concentrations described in Table 1-1 were used. Results are shown in Table 1-1.

### (Example 18: long pepper extract)

The tests were performed in the same manner as in Example 10, except that the hawthorn extract was changed to the long pepper extract (LONG PEPPER EXTRACT POWDER MF, product of MARUZEN PHARMACEUTICALS CO., LTD.) and concentrations described in Table 1-1 were used. Results are shown in Table 1-1.

### (Example 19: Quillaja extract)

The tests were performed in the same manner as in Example 10, except that the hawthorn extract was changed to the Quillaja extract (product of MARUZEN PHARMACEUTICALS CO., LTD.) and concentrations described in Table 1-1 were used. Results are shown in Table 1-1.

### (Example 20: Kouki extract)

The tests were performed in the same manner as in Example 10, except that the hawthorn extract was changed to the Kouki extract (KOUKI LEAF EXTRACT POWDER MF, product of MARUZEN PHARMACEUTICALS CO., LTD.) and concentrations described in Table 1-1 were used. Results are shown in Table 1-1.

### (Example 21: ginkgo extract)

The tests were performed in the same manner as in Example 10, except that the hawthorn extract was changed to the ginkgo extract (GINKGO LEAF DRY EXTRACT F, product of MARUZEN PHARMACEUTICALS CO., LTD.) and concentrations described in Table 1-1 were used. Results are shown in Table 1-1.

### (Example 22: oyster extract)

The tests were performed in the same manner as in Example 10, except that the hawthorn extract was changed to the oyster extract (OYSTER EXTRACT POWDER, product of MARUZEN PHARMACEUTICALS CO., LTD.) and concentrations described in Table 1-1 were used. Results are shown in Table 1-1.

### (Example 23: turmeric extract)

The tests were performed in the same manner as in Example 10, except that the hawthorn extract was changed to the turmeric extract (TURMERIC CONCENTRATED EXTRACT POWDER M, product of MARUZEN PHARMACEUTICALS CO., LTD.) and concentrations described in Table 1-1 were used. Results are shown in Table 1-1.

### (Example 24: chrysanthemum extract)

The tests were performed in the same manner as in Example 10, except that the hawthorn extract was changed to the chrysanthemum extract (CHRYSANTHEMUM FLOWER EXTRACT POWDER MF, product of MARUZEN PHARMACEUTICALS CO., LTD.) and concentrations described in Table 1-2 were used. Results are shown in Table 1-2.

### (Example 25: jujube extract)

The tests were performed in the same manner as in Example 10, except that the hawthorn extract was changed to the jujube extract (JUJUBE EXTRACT POWDER MF, product of MARUZEN PHARMACEUTICALS CO., LTD.) and concentrations described in Table 1-2 were used. Results are shown in Table 1-2.

### (Example 26: Chinese wolfberry extract)

The tests were performed in the same manner as in Example 10, except that the hawthorn extract was changed to the Chinese wolfberry extract (CHINESE WOLFBERRY EXTRACT POWDER MF, product of MARUZEN PHARMACEUTICALS CO., LTD.) and concentrations described in Table 1-2 were used. Results are shown in Table 1-2.

### (Example 27: chamomile extract)

The tests were performed in the same manner as in Example 10, except that the hawthorn extract was changed to the chamomile extract (CHAMOMILE EXTRACT POWDER MF, product of MARUZEN PHARMACEUTICALS CO., LTD.) and concentrations described in Table 1-2 were used. Results are shown in Table 1-2.

### (Example 28: Butcher's Broom extract)

The tests were performed in the same manner as in Example 10, except that the hawthorn extract was changed to the Butcher's Broom extract (BUTCHER'S BROOM EXTRACT LIQUID, product of MARUZEN PHARMACEUTICALS CO., LTD.) and concentrations described in Table 1-2 were used. Results are shown in Table 1-2.

### (Referential Example 5: positive control)

The test was performed in the same manner as in Example 10, except that the hawthorn extract was changed to Angiopoietin-1 (product of R&D Systems, Inc.) as a positive control and a concentration described in Table 1-2 was used. The Result is shown in Table 1-2.

**Table 1-1**

| | Test Example 2 | | |
|---|---|---|---|
| | Test Sample | | Evaluation |
| | Type | Concentration (µg/mL) | Activation rate of Tie2 (%) |
| Example 10 | Hawthorn extract (leaves) (HAWTHORN EXTRACT POWDER) | 50 | 112.2±3.5* |
| | | 100 | 126.0±4.7** |
| | | 200 | 128.0±3.0*** |
| | | 400 | 145.1±2.6*** |
| Example 11 | Starfruit extract (product of MARUZEN PHARMACEUTICALS CO., LTD.) | 200 | 130.5±3.8 |
| | | 400 | 140.7±1.7 |
| Example 12 | Shellflower extract (product of MARUZEN PHARMACEUTICALS CO., LTD.) | 200 | 107.2±2.9 |
| | | 400 | 124.0±5.1 |
| Example 13 | Lotus extract (product of MARUZEN PHARMACEUTICALS CO., LTD.) | 200 | 112.2±4.1 |
| | | 400 | 113.9±3.9 |
| Example 14 | Rooibos extract (product of MARUZEN PHARMACEUTICALS CO., LTD.) | 50 | 107.6±2.6 |
| | | 100 | 110.4±3.5 |
| | | 200 | 123.1±4.7 |
| | | 400 | 119.1±4.8 |
| Example 15 | Indian date extract (product of MARUZEN PHARMACEUTICALS CO., LTD.) | 12.5 | 121.1±8.7 |
| | | 25 | 144.5±7.9** |
| Example 16 | Chinese quince extract (product of MARUZEN PHARMACEUTICALS CO., LTD.) | 200 | 109.4±3.5 |
| | | 400 | 124.4±2.5** |
| Example 17 | Psidium guava extract (product of MARUZEN PHARMACEUTICALS CO., LTD.) | 200 | 100.2±4.1 |
| | | 400 | 114.0±3.7* |
| Example 18 | Long pepper extract (product of MARUZEN PHARMACEUTICALS CO., LTD.) | 200 | 117.0±4.6* |
| | | 400 | 108.8±1.3* |
| Example 19 | Quillaja extract (product of MARUZEN PHARMACEUTICALS CO., LTD.) | 6.25 | 151.4±9.7** |
| | | 12.5 | 158.3±3.6*** |
| Example 20 | Kouki extract (product of MARUZEN PHARMACEUTICALS CO., LTD.) | 200 | 117.4±4.7* |
| | | 400 | 129.8±1.2*** |
| Example 21 | Ginkgo extract (product of MARUZEN PHARMACEUTICALS CO., LTD.) | 100 | 134.4±4.0*** |
| | | 200 | 139.8±4.4** |
| Example 22 | Oyster extract (product of MARUZEN PHARMACEUTICALS CO., LTD.) | 200 | 134.9±8.7*** |
| | | 400 | 120.4±5.9* |
| Example 23 | Turmeric extract (product of MARUZEN PHARMACEUTICALS CO., LTD.) | 25 | 120.3±4.3* |
| | | 50 | 103.3±5.1 |

| | | | |
|---|---|---|---|
| Means ± S.E., n=4, *: *p*<0.05, **: *p*<0.01, ***: *p*<0.001 | | | |

**Table 1-2**

| | Test Example 2 | | |
|---|---|---|---|
| | Test Sample | | Evaluation |
| | Type | Concentration (µg/mL) | Activation rate of Tie2 (%) |
| Example 24 | Chrysanthemum extract (product of MARUZEN PHARMACEUTICALS CO., LTD.) | 200 | 118.1±1.3*** |
| | | 400 | 124.2±5.3** |
| Example 25 | Jujube extract (product of MARUZEN PHARMACEUTICALS CO., LTD.) | 200 | 105.6±3.2* |
| | | 400 | 104.7±4.4 |
| Example 26 | Chinese wolfberry extract (product of MARUZEN PHARMACEUTICALS CO., LTD.) | 100 | 115.7±2.1* |
| | | 400 | 114.8±2.5* |
| Example 27 | Chamomile extract (product of MARUZEN PHARMACEUTICALS CO., LTD.) | 200 | 114.8±1.8 |
| | | 400 | 113.9±4.7 |
| Example 28 | Butcher's Broom extract (product of MARUZEN PHARMACEUTICALS CO., LTD.) | 50 | 112.5±4.4 |
| | | 100 | 120.7±6.5 |
| | | 200 | 120.7±4.8 |
| Reference Example 5 | Positive control (Angiopoietin-1, product of R&D system) | 0.5 | 130.2±8.3* |

| | | | |
|---|---|---|---|
| Means ± S.E., n=4, *: *p*<0.05, **: *p*<0.01, ***: *p*<0.001 | | | |

### [Test Example 2: test result]

Test results of the Tie2 activating effect (immunoassay) now will be explained.

The Tie2 activating effect was evaluated using the immunoassay kit. The extracts used in Example were verified to phosphorylate and thus activate Tie2. Notably, when using DMSO as a negative control, Tie2 was not significantly phosphorylated. When using Anglopoletin-1 as a positive control in Reference Example 1, Tie2 was verified to be phosphorylated and thus activated. It has known that phosphorylation of Tie2 results in maturing, normalizing and stabilizing blood vessels, and inhibiting angiogenesis. Thus, it was suggested that the extracts used in Examples had a Tie2 phosphorylating effect, and thus inhibited angiogenesis, and induced maturation, normalization and stabilization of blood vessels.

### [Test Example 3: angiogenesis inhibitory effect test]

### (Example 29: hawthorn extract)

The pharmacological effect, i.e., angiogenesis inhibitory effect of the hawthorn extract was evaluated by measuring a decrease of the number of vascular branching by means of image analysis as follows.

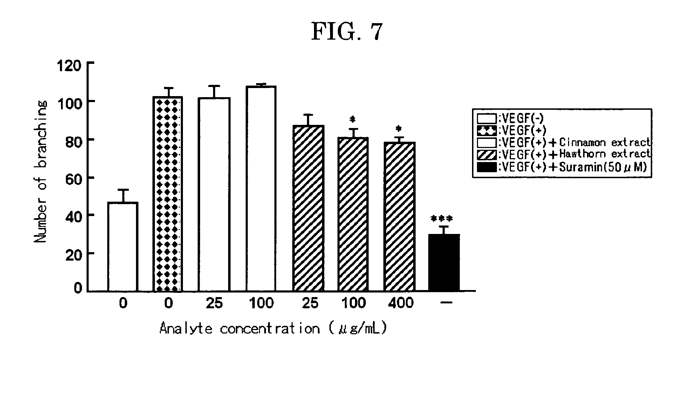

At first, human normal umbilical vein vascular endothelial cells were co-cultured with human normal dermal fibroblasts at a constant ratio using the angiogenesis kit (product of KURABO INDUSTRIES LTD.) to a proliferative state at the early stage of lumen formation. The test sample, the hawthorn extract (fruits) (HAWTHORN EXTRACT POWDER MF, product of MARUZEN PHARMACEUTICALS CO., LTD.), was added to media so as to have concentrations described in Table 2 in the presence of 10 ng/mL of vascular endothelial cell growth factor-A (VEGF-A) (product of KURABO INDUSTRIES LTD.), followed by incubation at 37°C and 5% CO₂. At the points of time after 4 days, 7 days, and 9 days of incubation, the media was replaced by media containing the hawthorn extract (fruits) (HAWTHORN EXTRACT POWDER MF, product of MARUZEN PHARMACEUTICALS CO., LTD.) having predetermined concentrations described in Table 2 in the presence of VEGF-A. Thereafter, cell layers in the wells were fixed in 70% by volume of ethanol. The CD31 (PECAM-1) in HUVEC cells was stained with the lumen staining kit (CD31, product of KURABO INDUSTRIES LTD.) The stained cells in the wells were photographed and counted for the number of luminal branching. Based on the thus-obtained number of luminal branching, the angiogenesis inhibitory effect was evaluated. Results are shown in Table 2 and FIG. 7.

### (Comparative Example 2: cinnamon extract)

The tests were performed in the same manner as in Examples 29, except that the hawthorn extract was changed to the cinnamon extract (CINNAMON EXTRACT POWDER MF, product of MARUZEN PHARMACEUTICALS CO., LTD.) and concentrations described in Table 2 were used. Results are shown in Table 2 and FIG. 7.

### (Reference Example 6: negative control)

The test was performed in the same manner as in Examples 29, except that the hawthorn extract was not used and the VEGF-A (+) was changed to VEGF-A (-), which served as a negative control. Results are shown in Table 2 and FIG. 7.

### (Reference Example 7: positive control)

The test was performed in the same manner as in Examples 29, except that the hawthorn extract was not used, which served as a positive control. Results are shown in Table 2 and FIG. 7.

### (Reference Example 8: Suramin)

The test was performed in the same manner as in Examples 29, except that the hawthorn extract was changed to Suramin (product of KURABO INDUSTRIES LTD. (appended to the angiogenesis kit)) (50 µM) which had been known as an angiogenesis inhibitor. Results are shown in Table 2 and FIG. 7.

**Table 2**

| | Test Example 3 | | | | | |
|---|---|---|---|---|---|---|
| | Vascular endothelial cell growth factor | | Test Sample | | Evaluation | |
| | Type | Concentration (ng/mL) | Type | Concentration (µg/mL) | Number of vascular branching | Angiogenesis inhibitory rate (%) |
| Example 29 | VEGF-A (+) | 10 | Hawthorn extract (fruits) (HAWTHORN EXTRACT POWDER (MF)) | 25 | 87.7±6.1 | 14.6 |
| | | | | 100 | 81.3±5.2* | 20.8* |
| | | | | 400 | 79.3±2.8* | 22.7* |
| Comparative Example 2 | VEGF-A (+) | 10 | Cinnamon extract (CINNAMON EXTRACT POWDER (MF)) | 25 | 102.3±6.6 | 0.3 |
| | | | | 100 | 108.3±1.9 | 5.5 |
| Reference Example 6 | VEGF-A (-) | 10 | - | 0 | 47.0±6.7 | - |
| Reference Example 7 | VEGF-A (+) | 10 | - | 0 | 102.7±5.5 | - |
| Reference Example 8 | VEGF-A (+) | 10 | Suramin (50µM) | - | 30.0±4.0*** | 70.8*** |

| | | | | | | |
|---|---|---|---|---|---|---|
| Means ± S.E., n=3, *: <0.05, ***: <0.001 | | | | | | |

### [Test Example 3: test result]

The angiogenesis inhibitory effect was evaluated based on the number of vascular branching. The hawthorn extract in Example 29 was verified to inhibit an increase of the number of vascular branching due to VEGF-A (+) in a concentration dependent manner (inhibitory rate: about 20%). Meanwhile, the cinnamon extract in Comparative Example 2 was not verified to have the angiogenesis inhibitory effect at the above-tested concentrations. Based on these results, the hawthorn extract was found to have a VEGF inhibitory effect, i.e., an effect inhibiting the action of VEGF, and thus have an effective angiogenesis inhibitory effect.

### Industrial Applicability

The Tie2 activator, the vascular endothelial growth factor (VEGF) inhibitor, the vascular maturing agent, the vascular normalizing agent, the vascular stabilizing agent and the angiogenesis inhibitor, and the pharmaceutical composition of the present invention have an excellent Tie2 activating effect, vascular endothelial growth factor (VEGF) inhibitory effect, angiogenesis inhibitory effect, vascular maturing effect, vascular normalizing effect, and vascular stabilizing effect, so that they can be widely used as pharmaceuticals for diseases with vascular lesions such as tumors, chronic rheumatoid arthritis, diabetic retinopathy, hyperlipemia or hypertension, and as safe preventive agents for the above-described diseases. It has been verified that the Tie2 activator, the vascular endothelial growth factor (VEGF) inhibitor, the vascular maturing agent, the vascular normalizing agent, the vascular stabilizing agent and the angiogenesis inhibitor of the present invention are not digested in a digestive tract. Therefore, they can be widely used as food and drink such as cosmetic food and drink or health food and drink.

### ITEMS

1. A Tie2 activator comprising, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.
2. An angiogenesis inhibitor comprising, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.
3. A vascular maturing agent, a vascular normalizing agent or a vascular stabilizing agent comprising, as an active ingredient, a hawthorn extract, a starfruit extract, a shellflower extract, a lotus extract, a rooibos extract, an Indian date extract, a Chinese quince extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, or a Butcher's Broom extract, or any combination thereof.
4. A vascular endothelial growth factor (VEGF) inhibitor comprising, as an active ingredient, a hawthorn extract.
5. A pharmaceutical composition comprising the Tie2 activator according to item 1, the angiogenesis inhibitor according to item 2, the vascular maturing agent, vascular normalizing agent or vascular stabilizing agent according to item 3, or the vascular endothelial growth factor (VEGF) inhibitor according to item 4, or any combination thereof.

## Claims

1. A Tie2 activator comprising, as an active ingredient, a starfruit extract, a rooibos extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, a Butcher's Broom extract, a hawthorn extract, a shellflower extract, a lotus extract, an Indian date extract, or a Chinese quince extract, or any combination thereof.

2. An angiogenesis inhibitor comprising, as an active ingredient, a starfruit extract, a rooibos extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, a Butcher's Broom extract, a hawthorn extract, a shellflower extract, a lotus extract, an Indian date extract, or a Chinese quince extract, or any combination thereof.

3. 3 A vascular maturing agent, a vascular normalizing agent or a vascular stabilizing agent comprising, as an active ingredient, a starfruit extract, a rooibos extract, a Psidium guava extract, a long pepper extract, a Quillaja extract, a Kouki extract, a ginkgo extract, an oyster extract, a turmeric extract, a chrysanthemum extract, a jujube extract, a Chinese wolfberry extract, a chamomile extract, a Butcher's Broom extract, a hawthorn extract, a shellflower extract, a lotus extract, an Indian date extract, or a Chinese quince extract, or any combination thereof.

4. A vascular endothelial growth factor (VEGF) inhibitor comprising, as an active ingredient, a starfruit extract.

5. A pharmaceutical composition comprising the Tie2 activator according to claim 1, the angiogenesis inhibitor according to claim 2, the vascular maturing agent, vascular normalizing agent or vascular stabilizing agent according to claim 3, or the vascular endothelial growth factor (VEGF) inhibitor according to claim 4, or any combination thereof.
